# EUROPEAN PATENT SPECIFICATION

(11) **EP 3 755 266 B1**
(45) Date of publication and mention of the grant of the patent: **03.04.2024**
(21) Application number: 19708747.1
(22) Date of filing: 19.02.2019
(51) Int. Cl.: A61F 2/04, A61F 2/90

(54) **DRAINAGE DEVICE**
DRAINAGEVORRICHTUNG
DISPOSITIF DE DRAINAGE

(30) Priority: 20.02.2018 US 201862632691 P
(43) Date of publication of application: 30.12.2020
(73) Proprietor: Boston Scientific Scimed, Inc., Maple Grove, MN 55311 (US)
(72) Inventor: FOLAN, Martyn G, Galway (IE); MOYLAN, Shane, Galway (IE); WALSH, Michael, Galway (IE); CURRAN, Darren, Galway (IE)
(74) Representative: Vossius & Partner Patentanwälte Rechtsanwälte mbB
(86) International application number: PCT/US2019/018493
(87) International publication number: WO 2019/164803

(56) References cited:
- WO-A1-2006/081448
- WO-A1-2015/195893
- US-A1- 2014 277 573

## Description

### CROSS REFERENCE TO RELATED APPLICATIONS

This application claims the benefit of priority under 35 U.S.C. §119 to United States Provisional Patent Application Serial No. 62/632,691, filed on February 20, 2018.

### FIELD

The present disclosure relates generally to the field of medical devices and establishing fluid communication between body lumens. In particular, the present disclosure relates to devices and methods for establishing a controlled flow or access passage between body lumens.

### BACKGROUND

The desire to establish access between two body lumens to create fluid communication from one to the other is present under various circumstances and conditions. A variety of medical devices (*e.g*., anastomotic devices, drainage stents, etc.) are able to establish open flow or access passages between body lumens. For example, during an endoscopic ultrasound-guided hepaticogastrostomy (EUS-HGS) procedure, an anastomotic or drainage device may be implanted to facilitate biliary drainage from the bile duct into the stomach or duodenum. To establish and maintain appropriate drainage, it is important that the anastomotic or drainage device does not substantially migrate within or between these body lumens.
WO 2015/195893 A1 discloses a tissue lumen stent that is provided with a body having an elongated tubular configuration and a foreshortened configuration. In the foreshortened configuration, downstream and upstream ends of the body expand radially into downstream and upstream flange structures, leaving a generally cylindrical saddle region therebetween. In some embodiments, the flange structures are non-symmetrical with respect to one another. Systems and methods of using the stents are also disclosed.
US 2014/0277573 A1 discloses implantable device embodiments, such as stents, and particularly esophageal stents, formed of a scaffolding structure. The scaffolding structure is formed of one or more strand elements arranged in a braided pattern. A covering may coat the scaffolding structure. A valve can be secured to the scaffolding structure and/or the covering. Anti -migration features may be formed by bends in the one or more strand elements. The bends forming the anti-migration features protrude outwardly away from an outer surface of the implantable device.

A variety of advantageous medical outcomes may be realized by the medical devices and/or methods of the present disclosure, which include, for example, an anastomotic or drainage device with one or more surface features to prevent or limit post-implantation migration of the properly positioned anastomotic or drainage device.

### SUMMARY

The invention is defined by the features of the claims. Embodiments not falling within the scope of the claims are not embodiments of the invention.

In one aspect, the present disclosure relates to a medical device, comprising an elongate tubular body formed of one or more woven filaments having a constrained configuration and an expanded configuration. In the expanded configuration, a proximal portion of the elongate tubular body may be expanded into a proximal retention member, a distal portion of the elongate tubular body may be expanded into a distal retention member and a cylindrical saddle region may extend between the proximal and distal retention members. The proximal retention member, distal retention member and cylindrical saddle region may define an open interior passage configured to permit flow therethrough. An outer surface of the cylindrical saddle region may include at least one surface feature. The proximal retention member may include a single or double-walled flange structure, and the distal retention member may include a flared flange structure. A diameter of the proximal and distal retention members may be larger than a diameter of the cylindrical saddle region. The cylindrical saddle region may include a constant outer diameter. The cylindrical saddle region may include a varying outer diameter. A surface of the distal retention member may be configured to contact a tissue wall of a second body lumen. A surface of the proximal retention member may be configured to contact a tissue wall of a first body lumen. The proximal retention member, distal retention member and cylindrical saddle region may be covered. The elongate tubular body may be formed of a plurality of the one or more filaments, and a proximal end of the elongate tubular body may include adjacent filaments formed into looped ends. The elongate tubular body may be formed of a plurality of the one or more filaments, and a proximal end of the elongate tubular body may include pointed free ends of the filaments. The surface feature may include, by way of non-limiting example, one or more circumferential rings, one or more helical rings and/or one more projections. An outer surface of the distal retention member may include at least one surface feature. The surface feature of the distal retention member may include, by way of non-limiting example, one or more circumferential rings, one or more helical rings and/or one or more projections. A retrieval loop may be threaded through the looped ends at the proximal end of the elongate tubular body. The retrieval loop may include at least one projection configured to be grasped by a medical device.

In another aspect, the present disclosure relates to a medical device, comprising an elongate tubular body formed of one or more woven filaments having a constrained configuration and an expanded configuration. In the expanded configuration, a proximal portion of the elongate tubular body may be expanded into a proximal retention member, a distal portion of the elongate tubular body may be expanded into a distal retention member and a cylindrical saddle region may extend between the proximal and distal retention members. The proximal retention member, distal retention member and cylindrical saddle region may define an open interior passage configured to permit flow therethrough. An outer surface of the distal retention member may include at least one surface feature. The proximal retention member may include a single or double-walled flange structure, and the distal retention member may include a flared-flange structure. A diameter of the proximal and distal retention members may be larger than a diameter of the cylindrical saddle region. The cylindrical saddle region may include a constant outer diameter. The cylindrical saddle region may include a varying outer diameter. A surface of the distal retention member may be configured to contact a tissue wall of a second body lumen. A surface of the proximal retention member may be configured to contact a tissue wall of a first body lumen. The proximal retention member, distal retention member and cylindrical saddle region may be covered. The elongate tubular body may be formed of a plurality of the one or more filaments, and a proximal end of the elongate tubular body may include adjacent filaments formed into looped ends. The elongate tubular body may be formed of a plurality of the one or more filaments, and a proximal end of the elongate tubular body may include pointed free ends of the filament. The surface feature of the distal retention member may include, by way of non-limiting example, one or more circumferential rings, one or more helical rings and/or one or more projections. A retrieval loop may be threaded through the looped ends at the proximal end of the elongate tubular body. The retrieval loop may include at least one projection configured to be grasped by a medical device.

In yet another aspect, the present disclosure relates to a method, comprising advancing a medical device into a first body lumen, wherein the medical device may include an elongate tubular body having a first constrained configuration and a second expanded configuration. The outer surface of the elongate tubular body may include at least one raised surface feature. The method may further include advancing the medical device into a second body lumen through an opening in the first body lumen. The method may further include expanding a distal portion of the elongate tubular body to the second expanded configuration, *e.g.*, such that a distal retention member of the elongate tubular body may be deployed within the second body lumen. The method may further include expanding a proximal portion of the elongate tubular body to the second configuration, such that a proximal retention member of the elongate tubular body may be deployed within the first body lumen.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 provides a perspective side view of a medical device, according to one embodiment of the present disclosure.
FIGS. 2A-2B provide perspective side views of a medical device, according to one embodiment of the present disclosure.
FIGS. 3A-3G provide perspective side views of a medical device, according to one embodiment of the present disclosure.
FIGS. 4A-4B provide perspective side views of a medical device, according to one embodiment of the present disclosure.
FIG. 5 provides a perspective side view of a medical device, according to one embodiment of the present disclosure.
FIG. 6 provides a perspective view of a medical device between a first body lumen and second body lumen, according to one embodiment of the present disclosure.

### DETAILED DESCRIPTION

The present disclosure is not limited to the particular embodiments described. The terminology used herein is for the purpose of describing particular embodiments only, and is not intended to be limiting beyond the scope of the appended claims. Unless otherwise defined, all technical terms used herein have the same meaning as commonly understood by one of ordinary skill in the art to which the disclosure belongs.

Although embodiments of the present disclosure are described with specific reference to medical devices (*e.g.*, anastomotic devices, stents, etc.) and methods for drainage of (or access to) the biliary system, it should be appreciated that such medical devices and methods may be used in a variety of medical procedures (*e.g*., external biliary drain conversion, enteroenterostomy, gastrojejumostomy, gastroduodenostomy and gastroileostomy, etc.) to establish and/or maintain a temporary or permanent open flow passage between or drainage from a variety of body organs, ducts, lumens, vessels, fistulas and spaces (*e.g.*, the dermis, stomach, duodenum, gallbladder, bladder, kidneys, walled-off pancreatic necrosis (WOPN), abscesses, etc.). Moreover, such medical devices are not limited to drainage, but may facilitate access to organs, vessels or body lumens for other purposes, such as creating a path to divert or bypass fluids or solids from one location to another, removing obstructions and/or delivering therapy, including non-invasive manipulation of the tissue within the organ and/or the introduction of pharmacological agents via the open flow passage.

As used herein, the singular forms "a," "an," and "the" are intended to include the plural forms as well, unless the context clearly indicates otherwise. It will be further understood that the terms "comprises" and/or "comprising," or "includes" and/or "including" when used herein, specify the presence of stated features, regions, steps elements and/or components, but do not preclude the presence or addition of one or more other features, regions, integers, steps, operations, elements, components and/or groups thereof.

As used herein, the term "distal" refers to the end farthest away from the medical professional when introducing a device into a patient, while the term "proximal" refers to the end closest to the medical professional when introducing a device into a patient.

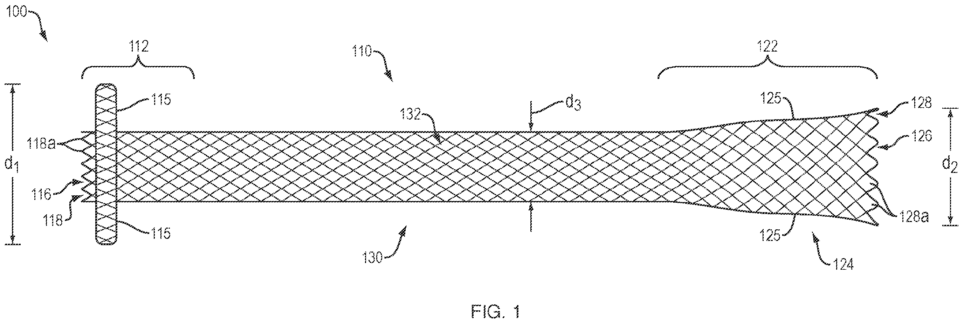

In various embodiments, the present disclosure relates to medical devices and methods for creating an open flow or access passage between two or more body lumens. Referring to FIG. 1, in one embodiment, a medical device 100 (*e.g.*, drainage stent) of the present disclosure may include an elongate tubular body 110 configured to move between a first (*e.g.*, constrained, collapsed, non-expanded) configuration and a second (*e.g.*, non-constrained, expanded) configuration. In the second configuration, a proximal portion 112 of the elongate tubular body 110 may form a proximal retention member 114 defining a first (*e.g.*, proximal) opening 116, and a distal portion 122 of the elongate tubular body 110 may form a distal retention member 124 defining a second (*e.g.*, distal) opening 126. A cylindrical saddle region 130 with a circumference and a longitudinal axis may extend between the proximal and distal retention members 114, 124 to define an open interior passage 132 (*e.g.*, channel, lumen, etc.) therebetween. A diameter d₃ of the cylindrical saddle region 130 may be greater than a diameter of the elongate tubular body 110 in the first configuration.

The proximal retention member 114 may extend radially from (*e.g*., perpendicular to a circumference of) the cylindrical saddle region 130 to define a surface 115 configured to atraumatically engage a (*e.g.*, inner) tissue wall of a first body lumen (*e.g.*, the stomach or duodenum). The distal retention member 124 may form a flared end (*e.g.*, flared flange structure), with an outer surface 125 of the flared end configured to atraumatically engage a (*e.g.*, inner) tissue wall of an adjacent or apposed second body lumen (*e.g.*, a biliary duct). In various embodiments, the surfaces 115, 125 of the proximal and distal retention members 114, 124 may prevent or limit movement/migration of the deployed medical device 100 within or between the first and second body lumens.

In one embodiment, an outer diameter d₁ of the proximal retention member 114 may be equal to an outer diameter d₂ of the distal retention member 124. The cylindrical saddle region 130 may include a constant outer diameter d₃ extending between the proximal and distal retention members 114, 124, wherein the diameter d₃ of the cylindrical saddle region is less than the diameters d₁ and d₂ of the proximal and distal retention members 114, 124. For example, outer diameters d₁ and d₂ may be approximately 7.0 mm to approximately 30 mm, and outer diameter d₃ may be approximately 3.0 mm to approximately 15.0 mm. In one or more embodiments, the proximal and distal retention members 114, 124 may include an outer diameter d₁ and d₂ that is as much as 75%-100% greater than an outer diameter d₃ of the cylindrical saddle region 130. In one embodiment, the cylindrical saddle region 130 may include a length of approximately 40-50 mm.

The elongate tubular body of any of the medical devices 100, 200, 300 depicted in FIGS. 1-6 may be formed of one or more woven, braided and/or knitted filaments (*e.g*., shape memory metals and polymers, nitinol wire, etc.). In various embodiments, the woven, braided and/or knitted filament(s) may further include a single filament woven upon itself, or multiple filaments woven together. In various embodiments, any of the woven, braided and/or knitted filament(s), which comprise the elongate tubular body, may include a variety of different cross-sectional shapes (*e.g.*, oval, round, flat, square, etc.).

In one embodiment, a distal end 128 of the elongate tubular body 110 may include an atraumatic configuration in which free ends of the one or more adjacent woven, braided or knitted filaments are bent and connected to form a series of atraumatic loop ends 128a. For example, each loop end 128a may be formed by mating and securing the adjacent free ends of the one or more filaments, *e.g*., by a weld, solder, adhesive, clamp, crimpable hypotube, or other suitable means as are known in the art. In another embodiment, a distal end 128 of the elongate tubular body may include an atraumatic configuration (*e.g*., looped ends) in which the one or more filaments are woven, braided or knitted on a mandrel. A distal end of the mandrel may include a plurality of posts, pins or pegs, around which the one or more filaments are wound. As will be understood by those of skill in the art, the atraumatic looped ends formed at the distal end 128 of the elongate tubular body 110 may represent an approximate midpoint of the one or more filaments, and the proximal end of the elongate tubular body may be formed by the free ends of each of the one or more filaments. In another embodiment, the proximal and distal ends of the elongate tubular body may include an atraumatic configuration (*e.g*., looped ends) in which the one or more filaments are woven, braided or knitted on a mandrel. The proximal and distal ends of the mandrel may include a plurality of posts, pins or pegs, around which the one or more filaments are wound. Free ends of the one or more filaments may be joined or connected (*e.g.*, using a suitable solder, glue, crimp, adhesive or resin, etc.) along an internal portion of elongate tubular body. For example, free ends of the same or different filaments may be joined along a body portion of the elongate tubular body between the proximal and distal retention members, *e.g*., along the cylindrical saddle region. Examples of suitable closed-loop ends formed from adjacent mated filament ends are further described in U.S. Patent Nos. 7,655,039; 8,109,988 and 9,788,979, and U.S. Patent Publication No. 2012/0101564.

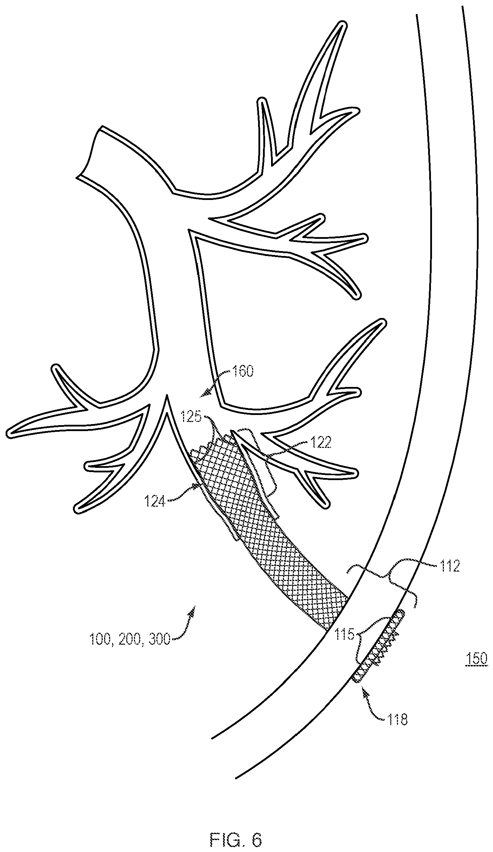

Although depicted schematically in FIG. 1, the loop ends 128a may include a variety of substantially angular configurations, including, by way of non-limiting example, semi-circular, semi-elliptical and other smoothly curved or substantially smoothly curved shapes. In one embodiment, a proximal end 118 of the elongate tubular body 110 may include free ends 118a of the one or more woven, braided or knitted filaments which are not connected, and instead form sharp or pointed free ends of the filament(s). As will be understood by those of skill in the art, and with reference to FIG. 6, the surface 115 of the proximal retention member 114 may atraumatically engage an inner tissue wall of a first body lumen such that the free ends 118a extend into the first body lumen and do not contact the tissue wall.

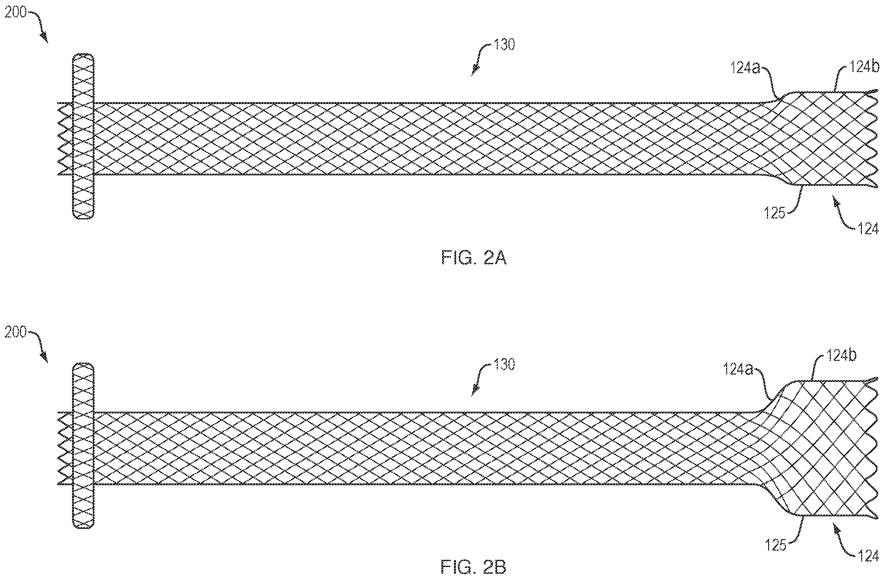

Referring to FIGS. 2A-2B, in one embodiment, a medical device 200 of the present disclosure may include the identical, or similar, configuration and elements as medical device 100, with the exception that the distal retention member 124 includes a first portion 124a that increases in diameter towards the distal end 128, and a second portion 124b extending distally beyond the first portion that is substantially parallel to the longitudinal axis of the cylindrical saddle region 130. In various embodiments, an outer surface 125 of the first and second portions 124a, 124b may atraumatically engage an inner surface of a second body lumen (*e.g.*, bile duct) to anchor the medical device 200.

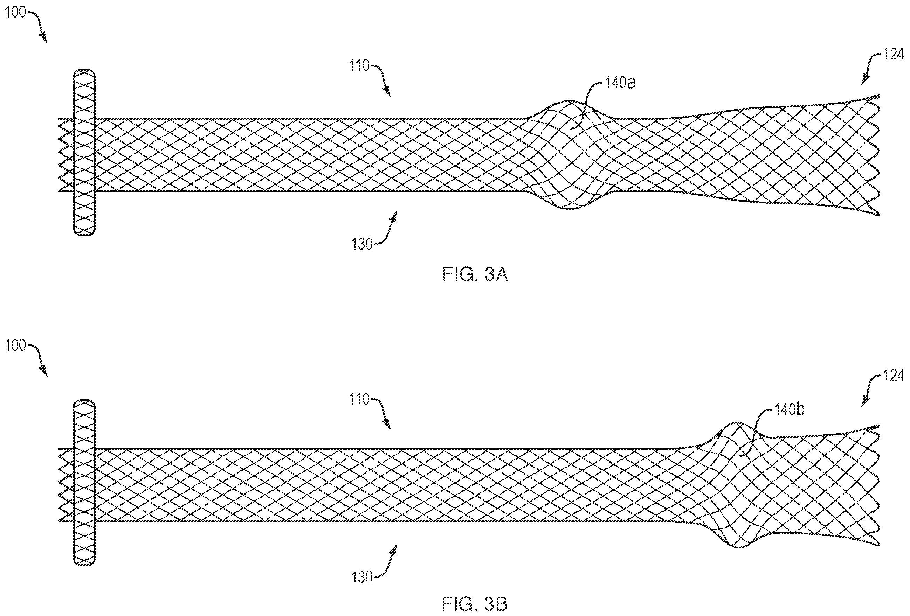

Referring to FIGS. 3A-3G, in various embodiments, any of the medical devices 100, 200 of the present disclosure may include one or more surface features (*e.g.*, anti-migration surface features) disposed along an outer surface of the elongate tubular body 110 and configured to atraumatically engage an inner wall of the second body lumen. In various embodiments, such surface features may be integrally formed from the one or more woven, braided and/or knitted filaments which form the elongate tubular body 110. In addition, or alternatively, such surface features may be attached or otherwise secured to the woven, braided and/or knitted filament(s) which form the elongate tubular body 110, *e.g.,* using a suitable weld, solder, glue, adhesive, resin or other bonding technique, as are commonly known in the art.

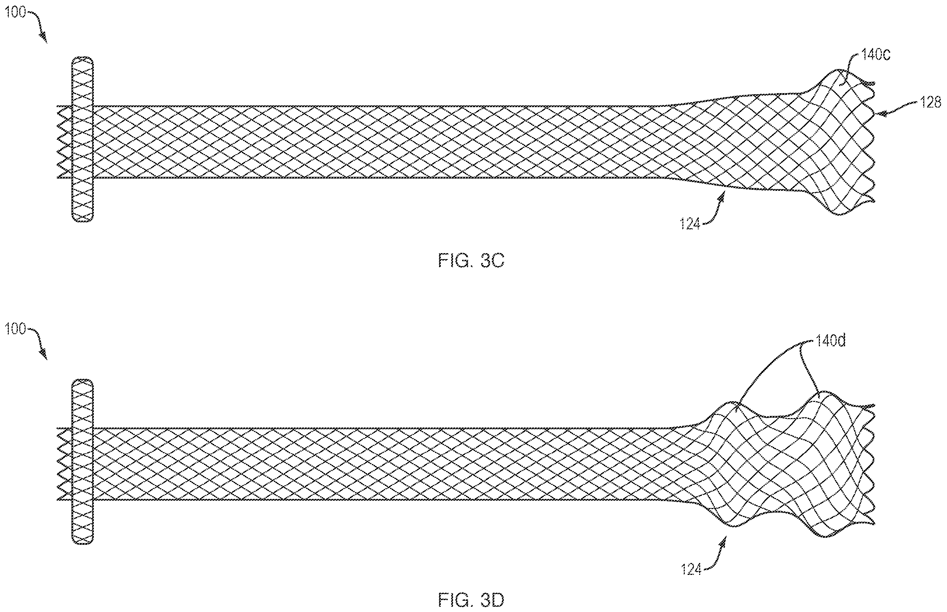

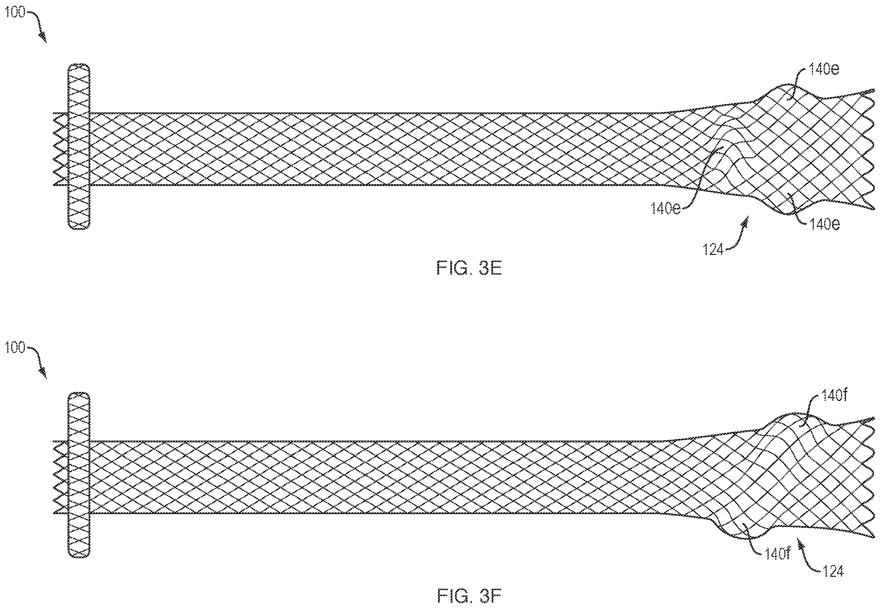

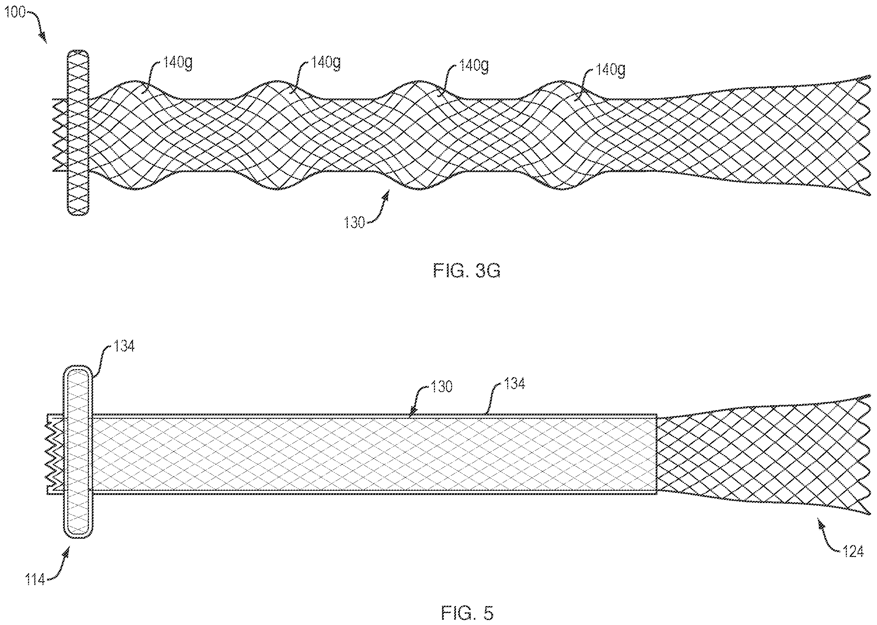

Referring to FIG. 3A, in one embodiment, a medical device 100, of the present disclosure may include a single surface feature 140a (*e.g.*, a ring or loop) extending around a full circumference of the cylindrical saddle region 130, and adjacent (*e.g*., proximal) to the distal retention member 124. Referring to FIG. 3B, in another embodiment, a medical device 100 of the present disclosure may include a single surface feature 140b extending around a full circumference of the elongate tubular body 110 at an approximate junction of the cylindrical saddle region 130 and the distal retention member 124. Referring to FIG. 3C, in another embodiment, a medical device 100 of the present disclosure may include a single surface feature 140c extending around a full circumference of the distal end 128 of the distal retention member 124. Referring to FIG. 3D, in another embodiment, a medical device 100 of the present disclosure may include a pair of spaced-apart surface features 140d extending around a full circumference of the distal retention member 124. Referring to FIG. 3E, in another embodiment, a medical device 100 of the present disclosure may include a series of bumps or projections 140e disposed in a symmetric or asymmetric pattern around a full circumference of the distal retention member 124. Referring to FIG. 3F, in another embodiment, a medical device 100 of the present disclosure may include a single surface feature 140f extending in a spiral or helical configuration around a full circumference of the distal retention member 124. Referring to FIG. 3G, in another embodiment, a medical device 100 of the present disclosure may include a series of surface features 140g (*e.g.*, undulations, bumps, etc.) extending in a symmetric or asymmetric pattern along a length of the cylindrical saddle region 130.

It should be appreciated that the medical devices of the present disclosure are not limited to the size, shape, number, arrangement and/or spacing of surface features 140a-g depicted in FIGS. 3A-3G, but may include surface features with any number of sizes, shapes, orientations, combinations and/or variations thereof. Similar surface features may be formed along any medical device of the present disclosure for anti-migration purposes, with or without the proximal and/or distal retention member configurations specific to the present disclosure.

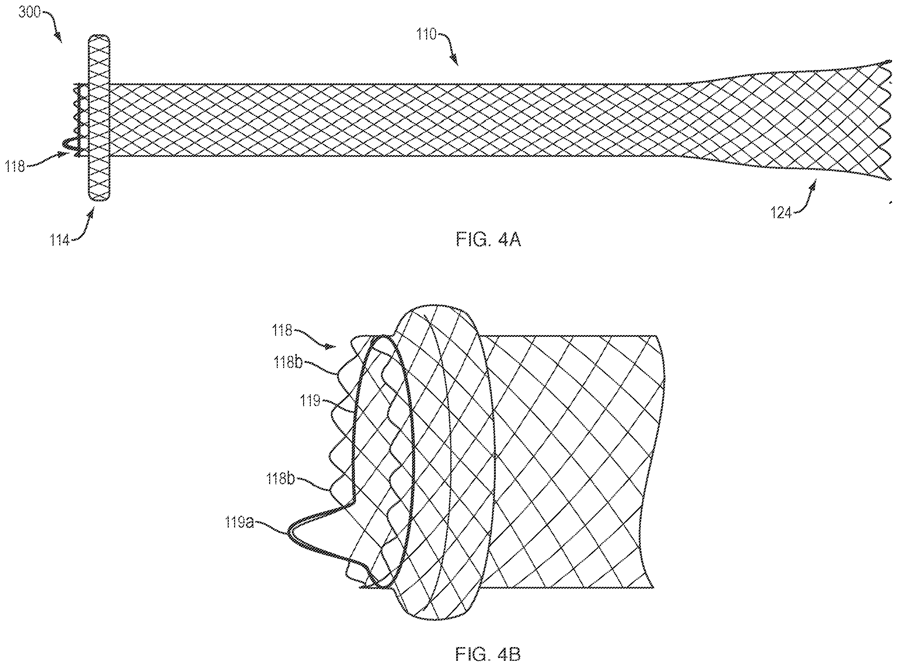

Referring to FIGS. 4A-4B, in one embodiment, a medical device 300 of the present disclosure may include the identical, or similar, configuration and elements as medical device 100 or 200, with the exception that the proximal end 118 of the elongate tubular body 110 includes an atraumatic configuration in which adjacent free ends of the one or more woven, braided or knitted filaments are bent and connected, or woven on a mandrel, to form a series of atraumatic loop ends 118b, *e.g.*, similar to the atraumatic loops 128a of FIG. 1 (discussed above). A repositioning or retrieval loop 119 (*e.g.*, braided-in retrieval loop) may be threaded through the atraumatic loop ends 118b around a circumference of the proximal end 118 of the elongate tubular body. The repositioning or retrieval loop 119 may further include a projection 119a configured to be engaged (*e.g.*, gripped, grasped, etc.) by a medical device (*e.g.*, forceps, etc.). In various embodiments, the repositioning or retrieval loop 119 may be formed from, or attached to, one or more of the woven, braided and/or knitted filaments which form the elongate tubular body 110, such that grasping and proximally retracting the projection 119a may urge the elongate tubular body 110 to move from the second configuration to the first configuration. In use and by way of example, a medical professional may grasp the projection 119a and exert the requisite proximal force on the retrieval loop 119 to properly position/reposition the proximal and distal retention members 114, 124 within the respective first and second body lumens. Alternatively, the medical professional may exert the requisite proximal force on the retrieval loop 119 to remove the medical device from the patient. Examples of suitable retrieval loops are described in U.S. Patent Publication No. 2016/0175124 and U.S. Patent No. 9,265,634.

In various embodiments, all or a portion of the elongate tubular body 110 of any of the medical devices 100, 200, 300 depicted in FIGS. 1-6 may further include a membrane, covering or coating on an inner and/or outer surface thereof to define a contiguous open interior passage configured for controlled flow (*e.g.*, body fluids, materials, and the like) and/or access therethrough. For example, referring to FIG. 5, in one embodiment, the proximal retention member 114 and cylindrical saddle region 130 may include a membrane, covering or coating 134, and the distal retention member 124 (*e.g.*, the portion disposed within the second body lumen) may be uncoated, *e.g.,* to facilitate tissue ingrowth to further reduce migration within or between the first and second bod lumens. In various embodiments, the coating may comprise a variety of non-degradable and biocompatible polymeric materials (*e.g.*, upon exposure to bodily fluids such as bile), including, for example, silicones, rubbers, polyethylenes, PVDF, Chronoflex^{®} and thermoplastic elastomers, such that the coating conforms to the medical device in the unexpanded and expanded configurations.

Referring to FIG. 6, in use and by way of example, a medical device 100, 200, 300 of the present disclosure may be disposed in the unexpanded configuration within the lumen of a delivery catheter which may include a tissue-penetrating element. A sharpened distal end of the tissue-penetrating element may be advanced through the tissue wall of a first body lumen 150 (*e.g.*, the stomach or duodenum) and into the opening of a second body lumen 160 (*e.g.*, the bile duct). Additionally, or alternatively, the tissue penetrating element may comprise a conductive element (*e.g*., halo wire cone with proximally extending arms) that is configured to receive heat or energy (*e.g.*, RF energy) for the purpose of creating openings.

In various embodiments, the tissue penetrating element may be advanced over a guidewire previously advanced through the first body lumen 150 and into the second body lumen 160 such that a distal end of the guidewire is disposed within the second body lumen 160. Alternatively, in the method above, a separate instrument with a sharpened distal tip may be advanced along the path above and into the second body lumen 160 to create a path. A guidewire may be put in place, or left in place, if used to guide the separate instrument, and the separate instrument withdrawn over the guidewire.

The distal portion 122 of the medical device 100, 200, 300 may then be further advanced distally beyond the lumen of the delivery catheter (which may or may not also include tissue-penetrating element), and/or an outer sheath of the delivery catheter may be retracted from the medical device, such that the distal retention member 124 is fully deployed within the second body lumen 160 and the surface 125 of the distal retention member 124 is placed in contact with the inner surface of the tissue wall of the second body lumen 160. The delivery catheter may then be further proximally retracted into the first body lumen 150, and the proximal portion 112 of the medical device 100, 200, 300 advanced distally beyond the lumen of the delivery catheter, and/or the outer sheath of the delivery catheter may be further retracted from about the medical device 100, 200, 300 such that the proximal retention member 114 is fully deployed within the first body lumen 150 and the surface 115 of the proximal retention member 114 is placed in contact with the inner surface of the tissue wall of the first body lumen 150.

The proximal retention member 114 of any of the medical devices 100, 200, 300 depicted in FIGS. 1-6 may include various configurations which extend radially at an angle from the longitudinal axis of the elongate tubular body that is not necessarily perpendicular to the elongate tubular body and/or the surfaces are not necessarily planar. In various embodiments, the angle of the proximal retention member 114 relative to the circumference and longitudinal axis of the cylindrical saddle region may assume other degrees (*e.g.*, 30, 45, 60, 75 degrees, etc.) or may change degrees along the length of the proximal retention member 114 creating inflection points in the retention member. For example, the proximal retention member 114 may extend outward towards an end of the elongate tubular body, back towards a center portion of the elongate tubular body, or change directions in some combination of both.

For example, the proximal retention member 114 may flare away from a longitudinal axis of the cylindrical saddle region 130 into a flange configuration when in the expanded configuration. The flange configuration may include at least first and second points of inflection that may define first and second segments of the flange. The first segment may extend from the first inflection point toward a center plane perpendicular to the longitudinal axis of the elongate tubular body, and the second segment may extend from the first inflection point away from the center plane. An angle of the first inflection point defined by the first segment and the cylindrical saddle region 130 may be at least as great as an opposing angle of the second inflection point defined by the first segment and the second segment.

As another example, the proximal flange may include at least first and second points of inflection that define first and second segments of the flange, wherein the second points of inflection may be further spaced radially from the longitudinal axis than the first points of inflection, and the second points of inflection may be closer than the first points of inflection to a center plane along the longitudinal axis. The proximal flange may touch planes that are parallel to the longitudinal axis, at least one plane each above and below the longitudinal axis, at at least two separate points along the parallel planes.

As yet a further example, the proximal flange configuration may include at least first and second points of inflection that define first and second segments of the flange. The first segment may extend from the first inflection point toward a center plane perpendicular to the longitudinal axis of the elongate tubular body 110, and the second segment may extend from the second inflection point away from the center plane. The intersection of the cylindrical saddle region 130 and the first segments may define the first inflection points, and the intersection of the first segments and second segments may define second inflection points. An angle of the first inflection points may be 90 degrees or less, and an opposing angle of the second inflection points may be 90 degrees or less.

Various embodiments, *e.g*., the medical devices 100, 200, 300 of the present disclosure, may include a single or double-walled flange as the proximal retention member 114 when the elongate tubular body 110 is in the expanded configuration. The walls of the proximal flange above and/or below the longitudinal axis may be symmetrical or may be asymmetrical. The walls of the proximal flange above and/or below the longitudinal axis may have multiple inflection points, as mentioned above, that define segments of the walls of the proximal flange that change direction as the walls extend radially away from the longitudinal axis (*e.g*., segments can extend radially parallel to, away from and/or toward, a radial center line of the body). The segments may extend along a straight line or may be curved, or may include a combination of straight lines and curves.

Although the medical devices 100, 200, 300 disclosed herein are generally depicted as including woven, knitted or braided filaments (*e.g*., nitinol, etc.), in various embodiments, the medical devices may include laser cut designs which may or may not change in length (*e.g.*, shorten) as the medical device moves from the first configuration to the second configuration. The medical devices in various configurations may be self-expanding or expandable such as balloon-expandable.

## Claims

1. A medical device (100; 200; 300), comprising:
an elongate tubular body (110) formed of one or more woven filaments having a constrained configuration,
the elongate tubular body (110) having an expanded configuration with a proximal portion (112) of the elongate tubular body (110) expanded into a proximal retention member (114), a distal portion (122) of the elongate tubular body (110) expanded into a distal retention member (124), and a cylindrical saddle region (130) extending between the proximal and distal retention members (114, 124);
wherein the proximal retention member (114), distal retention member (124) and cylindrical saddle region (130) define an open interior passage (132) configured to permit flow therethrough; and
wherein an outer surface of the distal retention member (124) includes at least one surface feature (140f) extending in a helical configuration around a full circumference of the distal retention member (124).

2. The medical device (100; 200; 300) of claim 1, wherein the proximal retention member (114) includes a single or double-walled flange structure, and the distal retention member (124) includes a flared flange structure.

3. The medical device (100; 200; 300) of any of claims 1-2, wherein a diameter (d₁, d₂) of the proximal and distal retention members (114, 124) is larger than a diameter (d₃) of the cylindrical saddle region (130).

4. The medical device (100; 200; 300) of any of claims 1-3, wherein the cylindrical saddle region (130) includes a constant outer diameter (d₃).

5. The medical device (100; 200; 300) of any of claims 1-4, wherein the cylindrical saddle region (130) includes a varying outer diameter (d₃).

6. The medical device (100; 200; 300) of any of claims 1-5, wherein a surface (125) of the distal retention member (124) is configured to contact a tissue wall of a second body lumen (160).

7. The medical device (100; 200; 300) of any of claims 1-6, wherein a surface of the proximal retention member (114) is configured to contact a tissue wall of a first body lumen (150).

8. The medical device (100; 200; 300) of any of claims 1-7, wherein the proximal retention member (114), distal retention member (124) and saddle region (130) are covered.

9. The medical device (100; 200; 300) of any of claims 1-8, wherein the elongate tubular body (110) is formed of a plurality of the one or more filaments, and a proximal end (118) of the elongate tubular body (110) includes adjacent filaments formed into looped ends (118b).

10. The medical device (100; 200; 300) of any of claims 1-8, wherein the elongate tubular body (110) is formed of a plurality of the one or more filaments, and a proximal end (118) of the elongate tubular body (110) includes pointed free ends (118a) of the filaments.

11. The medical device (100; 200; 300) of any of claims 1-10, wherein an outer surface of the cylindrical saddle region (130) includes at least one surface feature (140a; 140 b; 140g), wherein the surface feature (140a;140b; 140g) is selected from the group consisting of one or more circumferential rings, one or more helical rings and one or more projections.

12. The medical device (100; 200; 300) of any of claims 1-10, wherein an outer surface of the distal retention member (124) includes at least one further surface feature (140b; 140c; 140d; 140e; 140f).

13. The medical device (100; 200; 300) of claim 12, wherein the further surface feature (140c; 140d; 140e; 140f) of the distal retention member (124) is selected from the group consisting of one or more circumferential rings, one or more helical rings and one or more projections.

14. The medical device (100; 200; 300) of claim 9, further comprising a retrieval loop (119) threaded through the looped ends (118b) at the proximal end (118) of the elongate tubular body (110).

15. The medical device (100; 200; 300) of claim 14, wherein the retrieval loop (119) includes at least one projection (119a) configured to be grasped by a medical device.

## Patentansprüche

1. Medizinische Vorrichtung (100; 200; 300), mit:
einem länglichen rohrförmigen Körper (110), der aus einem oder mehreren gewebten Filamenten mit einer eingeschränkten Konfiguration ausgebildet ist,
wobei der längliche rohrförmige Körper (110) eine expandierte Konfiguration mit einem proximalen Abschnitt (112) des länglichen rohrförmigen Körpers (110), der zu einem proximalen Halteelement (114) expandiert ist, einem distalen Abschnitt (122) des länglichen rohrförmigen Körpers (110), der zu einem distalen Halteelement (124) expandiert ist, und einem sich zwischen dem proximalen und dem distalen Halteelement (114, 124) erstreckenden zylindrischen Sattelbereich (130) aufweist,
wobei das proximale Halteelement (114), das distale Halteelement (124) und der zylindrische Sattelbereich (130) einen offenen inneren Durchlass (132) definieren, der derart konfiguriert ist, dass er einen Durchfluss durch ihn hindurch erlaubt, und
wobei eine Außenfläche des distalen Halteelements (124) mindestens ein Oberflächenmerkmal (140f) aufweist, das sich in einer schraubenförmigen Konfiguration um den gesamten Umfang des distalen Halteelements (124) erstreckt.

2. Medizinische Vorrichtung (100; 200; 300) nach Anspruch 1, wobei das proximale Halteelement (114) eine einwandige oder eine doppelwandige Flanschstruktur aufweist und das distale Halteelement (124) die Struktur eines sich aufweitenden Flanschs aufweist.

3. Medizinische Vorrichtung (100; 200; 300) nach einem der Ansprüche 1 bis 2, wobei ein Durchmesser (d₁, d₂) des proximalen und des distalen Halteelements (114, 124) größer ist als ein Durchmesser (d₃) des zylindrischen Sattelbereichs (130).

4. Medizinische Vorrichtung (100; 200; 300) nach einem der Ansprüche 1 bis 3, wobei der zylindrische Sattelbereich (130) einen konstanten Außendurchmesser (d₃) aufweist.

5. Medizinische Vorrichtung (100; 200; 300) nach einem der Ansprüche 1 bis 4, wobei der zylindrische Sattelbereich (130) einen variablen Außendurchmesser (d₃) aufweist.

6. Medizinische Vorrichtung (100; 200; 300) nach einem der Ansprüche 1 bis 5, wobei eine Oberfläche (125) des distalen Halteelements (124) dafür konfiguriert ist, mit einer Gewebewand eines zweiten Körperlumens (160) in Kontakt zu kommen.

7. Medizinische Vorrichtung (100; 200; 300) nach einem der Ansprüche 1 bis 6, wobei eine Oberfläche des proximalen Halteelements (114) dafür konfiguriert ist, mit einer Gewebewand eines ersten Körperlumens (150) in Kontakt zu kommen.

8. Medizinische Vorrichtung (100; 200; 300) nach einem der Ansprüche 1 bis 7, wobei das proximale Halteelement (114), das distale Halteelement (124) und der Sattelbereich (130) abgedeckt sind.

9. Medizinische Vorrichtung (100; 200; 300) nach einem der Ansprüche 1 bis 8, wobei der längliche rohrförmige Körper (110) aus einer Menge von dem einen oder den mehreren Filamenten gebildet ist und ein proximales Ende (118) des länglichen rohrförmigen Körpers (110) benachbarte Filamente aufweist, die als schlaufenförmige Enden (118b) ausgebildet sind.

10. Medizinische Vorrichtung (100; 200; 300) nach einem der Ansprüche 1 bis 8, wobei der längliche rohrförmige Körper (110) aus einer Menge von dem einen oder den mehreren Filamenten gebildet ist und ein proximales Ende (118) des länglichen rohrförmigen Körpers (110) spitze freie Enden (118a) der Filamente aufweist.

11. Medizinische Vorrichtung (100; 200; 300) nach einem der Ansprüche 1 bis 10, wobei eine Außenfläche des zylindrischen Sattelbereichs (130) mindestens ein Oberflächenmerkmal (140a; 140b; 140g) aufweist, wobei das Oberflächenmerkmal (140a; 140b; 140g) ausgewählt ist aus der Gruppe bestehend aus einem oder mehreren umlaufenden Ringen, einem oder mehreren spiralförmigen Ringen und einem oder mehreren Vorsprüngen.

12. Medizinische Vorrichtung (100; 200; 300) nach einem der Ansprüche 1 bis 10, wobei eine Außenfläche des distalen Halteelements (124) mindestens ein weiteres Oberflächenmerkmal (140b; 140c; 140d; 140e; 140f) aufweist.

13. Medizinische Vorrichtung (100; 200; 300) nach Anspruch 12, wobei das weitere Oberflächenmerkmal (140c; 140d; 140e; 140f) des distalen Halteelements (124) ausgewählt ist aus der Gruppe bestehend aus einem oder mehreren umlaufenden Ringen, einem oder mehreren spiralförmigen Ringen und einem oder mehreren Vorsprüngen.

14. Medizinische Vorrichtung (100; 200; 300) nach Anspruch 9, ferner mit einer Rückführungsschlaufe (119), die durch die schlaufenförmigen Enden (118b) am proximalen Ende (118) des länglichen rohrförmigen Körpers (110) gefädelt ist.

15. Medizinische Vorrichtung (100; 200; 300) nach Anspruch 14, wobei die Rückführungsschlaufe (119) mindestens einen Vorsprung (119a) aufweist, der derart konfiguriert ist, dass eine medizinische Vorrichtung diese fassen kann.

## Revendications

1. Dispositif médical (100 ; 200 ; 300), comprenant :
un corps tubulaire allongé (110) formé d'un ou plusieurs filaments tissés ayant une configuration contrainte,
le corps tubulaire allongé (110) ayant une configuration déployée avec une partie proximale (112) du corps tubulaire allongé (110) déployée dans un élément de rétention proximal (114), une partie distale (122) du corps allongé tubulaire (110) déployée dans un élément de rétention distal (124), et une région de selle cylindrique (130) s'étendant entre les éléments de rétention proximal et distal (114, 124) ;
dans lequel l'élément de rétention proximal (114), l'élément de rétention distal (124) et la région de selle cylindrique (130) définissent un passage intérieur ouvert (132) configuré pour permettre un écoulement à travers lui ; et
dans lequel une surface externe de l'élément de rétention distal (124) inclut au moins une caractéristique de surface (140f) s'étendant dans une configuration hélicoïdale autour d'une circonférence complète de l'élément de rétention distal (124).

2. Dispositif médical (100 ; 200 ; 300) selon la revendication 1, dans lequel l'élément de rétention proximal (114) inclut une structure de bride à paroi unique ou à double paroi, et l'élément de rétention distal (124) inclut une structure de bride évasée.

3. Dispositif médical (100 ; 200 ; 300) selon l'une quelconque des revendications 1 à 2, dans lequel un diamètre (d₁, d₂) des éléments de rétention proximal et distal (114, 124) est supérieur à un diamètre (d₃) de la région de selle cylindrique (130).

4. Dispositif médical (100 ; 200 ; 300) selon l'une quelconque des revendications 1 à 3, dans lequel la région de selle cylindrique (130) inclut un diamètre externe constant (d₃).

5. Dispositif médical (100 ; 200 ; 300) selon l'une quelconque des revendications 1 à 4, dans lequel la région de selle cylindrique (130) inclut un diamètre externe variable (d₃).

6. Dispositif médical (100 ; 200 ; 300) selon l'une quelconque des revendications 1 à 5, dans lequel une surface (125) de l'élément de rétention distal (124) est configurée pour entrer en contact avec une paroi tissulaire d'une deuxième lumière corporelle (160).

7. Dispositif médical (100 ; 200 ; 300) selon l'une quelconque des revendications 1 à 6, dans lequel une surface de l'élément de rétention proximal (114) est configurée pour entrer en contact avec une paroi tissulaire d'une première lumière corporelle (150).

8. Dispositif médical (100 ; 200 ; 300) selon l'une quelconque des revendications 1 à 7, dans lequel l'élément de rétention proximal (114), l'élément de rétention distal (124) et la région de selle (130) sont recouverts.

9. Dispositif médical (100 ; 200 ; 300) selon l'une quelconque des revendications 1 à 8, dans lequel le corps tubulaire allongé (110) est formé d'une pluralité des un ou plusieurs filaments, et une extrémité proximale (118) du corps tubulaire allongé (110) inclut des filaments adjacents et formés en extrémités en boucle (118b).

10. Dispositif médical (100 ; 200 ; 300) selon l'une quelconque des revendications 1 à 8, dans lequel le corps tubulaire allongé (110) est formé d'une pluralité des un ou plusieurs filaments, et une extrémité proximale (118) du corps tubulaire allongé (110) inclut des extrémités libres pointues (118a) des filaments.

11. Dispositif médical (100 ; 200 ; 300) selon l'une quelconque des revendications 1 à 10, dans lequel une surface externe de la région de selle cylindrique (130) inclut au moins une caractéristique de surface (140a ; 140b ; 140g), dans lequel la caractéristique de surface (140a ; 140b ; 140g) est choisie dans le groupe consistant en un ou plusieurs anneaux circonférentiels, un ou plusieurs anneaux hélicoïdaux et une ou plusieurs saillies.

12. Dispositif médical (100 ; 200 ; 300) selon l'une quelconque des revendications 1 à 10, dans lequel une surface externe de l'élément de rétention distal (124) inclut au moins une autre caractéristique de surface (140b ; 140c ; 140d ; 140e ; 140f).

13. Dispositif médical (100 ; 200 ; 300) selon la revendication 12, dans lequel l'autre caractéristique de surface (140c ; 140d ; 140e ; 140f) de l'élément de rétention distal (124) est choisie dans le groupe consistant en un ou plusieurs anneaux circonférentiels, un ou plusieurs anneaux hélicoïdaux et une ou plusieurs saillies.

14. Dispositif médical (100 ; 200 ; 300) selon la revendication 9, comprenant en outre une boucle de récupération (119) filetée à travers les extrémités en boucle (118b) au niveau de l'extrémité proximale (118) du corps tubulaire allongé (110).

15. Dispositif médical (100 ; 200 ; 300) selon la revendication 14, dans lequel la boucle de récupération (119) inclut au moins une saillie (119a) configurée pour être saisie par un dispositif médical.
